## Europäisches Patentamt
## European Patent Office
## Office européen des brevets

(19)

(11) Veröffentlichungsnummer: **0 054 562**
**B1**

(12)

# EUROPÄISCHE PATENTSCHRIFT

(45) Veröffentlichungstag der Patentschrift:
**09.05.84**

(21) Anmeldenummer: **81901807.8**

(22) Anmeldetag: **11.06.81**

(86) Internationale Anmeldenummer:
**PCT/DE 81/00089**

(87) Internationale Veröffentlichungsnummer:
**WO 82/00035 (07.01.82 Gazette 82/1)**

(51) Int. Cl.³: **C 09 B 61/00, A 61 K 7/00,
A 23 L 1/275, D 06 P 1/34**

(54) **NATÜRLICHER GENIESSBARER FARBSTOFF.**

(30) Priorität: **20.06.80 DE 3023178**

(43) Veröffentlichungstag der Anmeldung:
**30.06.82 Patentblatt 82/26**

(45) Bekanntmachung des Hinweises auf die Patenterteilung:
**09.05.84 Patentblatt 84/19**

(84) Benannte Vertragsstaaten:
**AT CH DE FR GB LI NL SE**

(56) Entgegenhaltungen:
**DE - A - 761 567
DE - C - 2 112
DE - C - 70 682
US - A - 4 204 043**

**Chemical Abstracts, vol. 79, 6 March 1973, (Columbus,
Ohio, US), see page 288, abstract 124920a, JP, A,
7317825, 14 July 1971, Taito Co., Ltd
Chemical Abstracts, vol. 72, 1970, (Columbus, Ohio, US),
Yoshikura, Kazuko u.a. "Anthocyanins of the balck
soybean", see page 93, abstract 63600c, Eiyo To
Shokuryo 1969, 22(6), 367-70 (JA)
Brockhaus Enzyklopädie 1967, 3. Band, S.82
Meyers Enz. Lexikon 1978, Band 4, S.445**

(73) Patentinhaber: **Dragoco Gerberding & Co. GmbH,
Dragocostrasse 1, D-3450 Holzminden (DE)**

(72) Erfinder: **HOFFMANN, Paul, Wiesenweg 35,
D-3450 Holzminden (DE)**

(74) Vertreter: **Deufel, Paul, Dr. et al, Patentanwälte
Müller-Boré, Deufel, Schön, Hertel, Lewald, Otto
Isartorplatz 6 Postfach 26 02 47, D-8000 München 26 (DE)**

## Beschreibung

Gegenwärtig werden für Lebensmittel, Genussmittel, Getränke, pharmazeutische und kosmetische Erzeugnisse überwiegend synthetische Farbstoffe verwendet. Da einige dieser Farbstoffe physiologisch nicht unbedenklich sind und der Verbraucher umweltbewusster geworden ist, geht der Trend zu natürlichen Farbstoffen. Dieser Trend wird auch gefördert durch die Gesetzgebung und Rechtsprechung auf dem Gebiet des Lebensmittelrechts in allen Ländern der Erde.

Soweit für die oben genannten Erzeugnisse bereits natürliche Farbstoffe aus Blüten oder Früchten, z.B. aus Hibiskusblüten oder aus Holunderbeeren, verwendet werden, sind diese durch Extraktionsverfahren gewonnen worden und weisen den Nachteil auf, dass sie nicht ausreichend thermo- und lichtstabil sind und sich nur schwer standardisieren lassen.

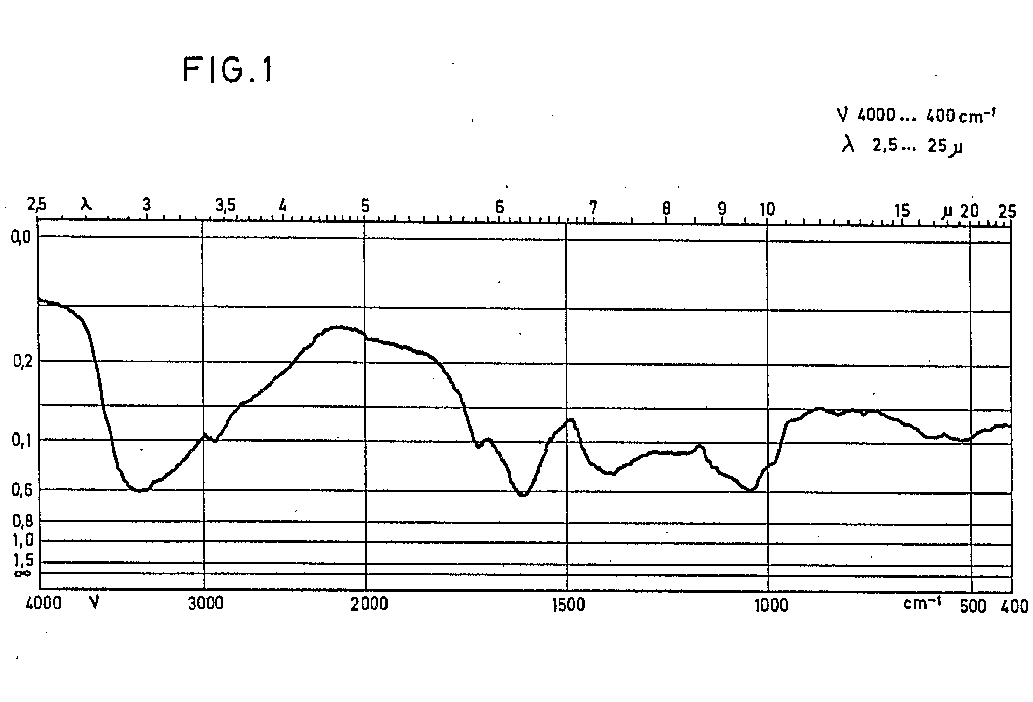

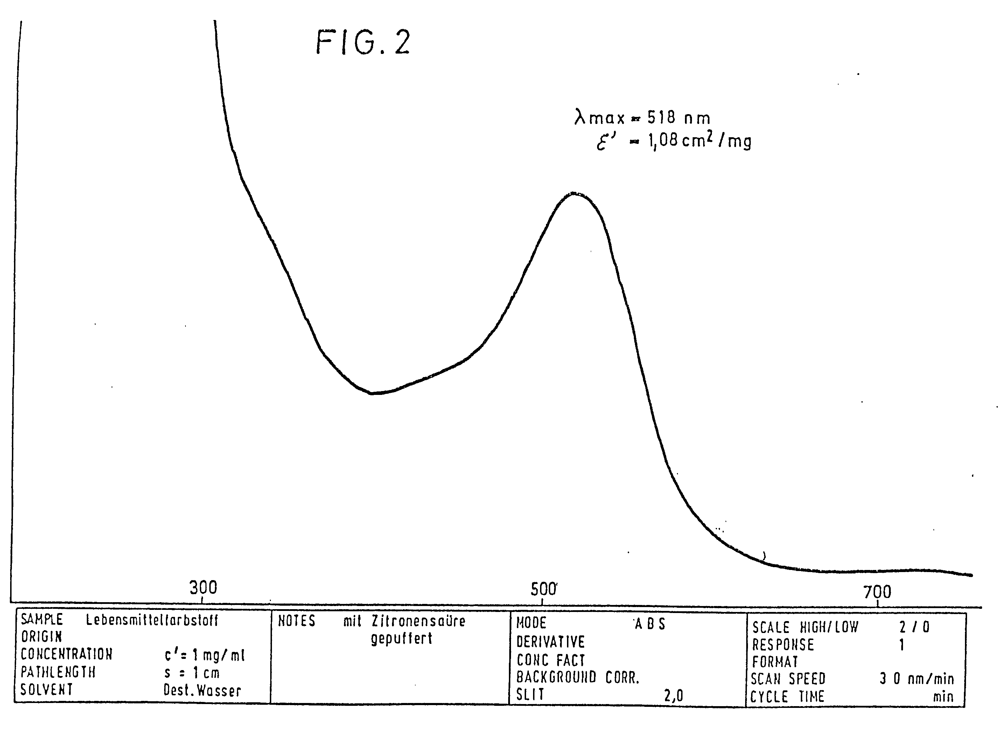

Es wurde nun überraschenderweise gefunden, dass der aus natürlichen Grundstoffen erhältliche Farbstoff gemäss den in Figur 1 und 2 dargestellten IR-bzw. UV-Spektren die vorstehend beschriebenen Nachteile beseitigt. Versuche haben ergeben, dass mit diesem Farbstoff bearbeitete Erzeugnisse auch nach einjähriger Lagerung bei Tageslicht und Zimmertemperatur keinerlei farbliche Änderungen aufweisen. Auch höhere Temperaturen, wie sie beispielsweise in den Tropen auftreten, beeinflussen die Farbqualität nicht. Der Farbstoff zeigt bei auf diesem Gebiet normaler Verdünnung ein kräftiges rot, und die Färbung mit ihm können, je nach Konzentration, von rosa bis tief dunkelblau-rot eingestellt werden, wie die folgende Tabelle zeigt:

Tabelle I

Färbung 0,1%iger Lösungen des Farbstoffes in $H_2O$ (Farbstoff mit Zitronensäure extrahiert)
pH 1,0: schönes kräftiges rot ohne Lilastich (mit HCl angesäuert)
pH 3,0: rot mit leichtem Lilastich
pH 4,0: rot mit leichtem Lilastich, aber kräftiger als bei pH 3,0
pH 5,6: rot mit Lilastich, auberginefarben
pH 7,0: braun-grün (mit $NH_4OH$ eingestellt)
pH 9,0: grün
pH 12,0: grün mit Gelbstich (mit NaOH eingestellt)

Nach dem Alkalisch stellen und erneutem Ansäuren lässt die Farbintensität nach und ändert sich in orange (pH 4,0).

Eine 10%ige Farbstofflösung ist kräftig auberginefarben (pH 5,5).

Für Färbungszwecke auf dem Lebensmittelsektor eignen sich folgende Konzentrationen:

blass rosa: 0,05%ige Lösung pH 4,5
kräftig rot: 0,35%ige Lösung pH 4,5
tief rot: 0,55%ige Lösung pH 4,5

Eine auf 50 Brix eingeengte und dann sprühgetrocknete Lösung ergibt ein Pulver von fast schwarz-violetter Färbung.

Es ist bemerkenswert, dass die Farbstofflösung auch bei normalen Atmosphärendruck und 100°C eingeengt werden kann, ohne dass der Farbstoff zerstört wird.

Das Verfahren zur Herstellung des Farbstoffes geht von farbigen Bohnen der Gattung Phaseolus, insbesondere von den schwarz gefärbten Kulturformen der gewöhnlichen Bohnen Phaseolus vulgaris aus, die in Südamerika eines der Grundnahrungsmittel der einheimischen Bevölkerung sind. Dieses Ausgangsmaterial ist gegenüber anderen natürlichen Farbstoffgrundlagen oder dem Ausgangsmaterial für synthetische Farbstoffe erheblich billiger. Es können als Ausgangsmaterial die Schalen aller gefärbten Varietäten der Gattung Phaseolus verwendet werden. Neben der schon erwähnten Phaseolus vulgaris seien noch die Feuerbohnen (Phaseolus coccineus) und Phaseolus caracalla besonders genannt.

Das Verfahren weist folgende Grundschritte auf:

a) Die Schalen der erwähnten schwarzen ungekochten aber zweckmässig kurz eingeweichten Bohnen oder deren Pulver werden mit soviel schwach saurem Wasser, vorzugsweise der wässrigen Lösung einer Oxycarbonsäure, dass eine gut rührfähige Suspension entsteht, einige Zeit gekocht. Der pH-Wert sollte nach 10- bis 15-minütigem Kochen auf etwa 5 bis 5,5 stehen.

b) Nach Abkühlen auf die für die nachfolgende Verfahrensstufe zweckmässige Temperatur werden die Zellwände der Bohnenschalen wenigstens teilweise zerstört, um den Farbstoff freizusetzen. Die Zellwandzerstörung erfolgt in an sich bekannter Weise durch enzymatischen Abbau, Vermahlen unter tiefen Temperaturen, gegebenenfalls unter Zusatz von Trockenreis und/oder feinteiligem Aluminiumoxid oder durch Autoklavieren unter erhöhtem Druck und erhöhter Temperatur, oder durch Hochdruckextraktion mit überkritischen Gasen, z.B. $CO_2$, oder durch Flüssig-flüssig-Extraktion mit geeigneten Lösungsmitteln, z.B. Methanol.

c) Die erhaltene Suspension wird wieder auf einen pH-Wert von etwa 4 bis 4,5 gebracht, wozu erforderlichenfalls etwas Säure zugegeben wird. Es ist vorteilhaft, dann kurz aufzukochen, was jedenfalls bei enzymatischem Abbau zur Enzymzerstörung angebracht ist, aber auch dazu dient, den Farbstoff weitgehendst aus den Zellen herauszulösen.

d) Die erhaltene Suspension wird in Schalenrückstände einerseits und die Farbstofflösung andererseits getrennt, was durch Absieben, Filtrieren oder Zentrifugieren und dergleichen erfolgen kann und

e) die Farbstofflösung wird in an sich bekannter Weise eingeengt, gegebenenfalls bis zur Trockne, was durch Konzentration, insbesondere im Vakuum, oder durch Sprühtrocknen erfolgen kann.

Der Verfahrensschritt a) kann auch mit ganzen Bohnen, die gegebenenfalls gekocht und/oder zerkleinert sind, durchgeführt werden. Das Ergebnis ändert sich dadurch nicht grundsätzlich. Bei enzymatischem Zellwandabbau werden, ebenso wie beim Abbau durch Autoklavieren, bevorzugt die Zellwände der Bohnenschalen angegriffen. Die grössere zu verarbeitende Menge erfordert jedoch zweckmässig ein zweifaches Aufkochen in der Stufe a), wodurch das Verfahren aufwendiger wird. Bei Zellzerstörung durch Vermahlen ganzer Bohnen ist natürlich die Farbstoffkonzentration im Gesamtbrei sehr viel geringer und die Aufkoch- und Extraktionsstufen müssen mehrfach wiederholt werden, was insgesamt ein weniger elegantes Verfahren bietet. Da mechanische Schälmaschinen für Hülsenfrüchte bekannt sind und die beim Schälen anfallenden Bohnenkerne für anderweitige Verwendung, z.B. als Bohnenmus zur Verfügung stehen, ist die Verwendung von Bohnenschalen als Ausgangsmaterial hochgradig bevorzugt. Beim Schälen soll auch die dicht unter der Schale sitzende oberste Schicht der Bohnen noch mit abgenommen werden, da diese ebenfalls noch Farbstoff enthält. Übliche Schälmaschinen ziehen etwa 10% der Masse des Kernes mit ab. Als Ausgangsmaterial eignen sich auch sehr gut der als Rückstand beim Entschälen schwarzer Bohnen für weisses Bohnenmus enfallende Schalenstaub. Als Entschälmaschine hat sich die vertikale Schleifmaschine für Reis, Getreide und Hülsenfrüchte, Typ DSRD, der Firma Bühler-Miag bewährt.

Zum Ansäuern in den Stufen a) und c) eignen sich insbesondere schwächere organische Säuren, wobei Oxycarbonsäuren, insbesondere Zitronensäure bevorzugt sind. Zu nennen ist auch Ascorbinsäure als gut geeignet.

Der entscheidende Schritt des vorliegenden Verfahrens ist die Zerstörung der Zellwände der Bohnenschalen in einem Ausmass, das dem Farbstoff den Durchtritt durch die Zellwände gestattet. Die Verwendung von Säureamylase bei diesem Verfahrensschritt hat den weiteren Vorteil, dass die von Stärkepartikeln umgebenen Zellwände leicht und schnell hinreichend aufgebrochen werden und somit der in der Zelle enthaltene Farbstoff in besonders guter Ausbeute isoliert werden kann.

Der verfahrensgemäss hergestellte Farbstoff kann sowohl als Lösung als auch durch bekannte Verfahren auf eine vom Anwender gewünschte Trockensubstanz, z.B. 60%, eingeengt oder pulverförmig auf den Markt gebracht werden.

Die Intensität der Farbgebung kann bei allen einzufärbenden Produkten in einfacher Weise durch Änderung der Farbstoffmenge variiert, aber auch wie in Tabelle I gezeigt durch das pH beeinflusst werden.

Die Konstitution des Farbstoffes ist durch die beigefügten Spektren definiert. Die wesentlichen physikalisch-chemischen Eigenschaften, insbesondere die Wärme- und Lichtbeständigkeit unterscheiden den erfindungsgemässen Farbstoff von den bekannten Anthocyaninen, so dass angenommen werden muss, dass es sich um einen speziellen Anthocyanin-Farbstoffkomplex handelt, der durch die spezielle Herstellungsweise modifiziert und stabilisiert wird.

Die folgenden Beispiele erläutern die Erfindung. Bei den als Ausgangsmaterial eingesetzten schwarzen Bohnen handelte es sich um solche der Gattung Phaseolus vulgaris.

Beispiel 1

Dieses Beispiel zeigt die Gewinnung des Farbstoffes aus den Schalen schwarzer Bohnen.

1000 g schwarze Bohnen werden mechanisch geschält, wobei 250 g Schale gewonnen werden, die für das weitere Verfahren benötigt werden. Das rückständige Bohnenmus wird abgesondert und steht für anderweitige Verwendung zur Verfügung. Beim Schälen wird auch noch die oberste, deutlich gefärbte Schicht des Bohnenkernes selbst mitabgeschliffen. Zum Schälen wird eine handelsübliche Labormaschine, wie sie für das Schälen von Hülsenfrüchten bekannt sind, eingesetzt.

Die erhaltenen 250 g Schalen werden mit einem Gemisch aus 2 l Wasser und 0,5 g pulverisierter kristallierter Zitronensäure 10 Minuten lang gekocht, wodurch der pH-Wert auf 5 bis 5,5 gebracht wird.

Das erhaltene Gemisch wird auf eine Temperatur von 53 bis 56°C abgekühlt, um die optimalen pH- und Temperaturwerte für die nachfolgende Enzymbehandlung zu liefern.

Dem Gemisch wird unter Beibehaltung der Temperatur 0,5 g Säureamylase (Röhm Pharma GmbH): hinzugefügt, wobei die Inkubationszeit 30 Minuten beträgt.

Dann werden dem Gemisch 14 g Zitronensäure 50%ig zugesetzt und das ganze anschliessend 5 Minuten lang gekocht, um die Enzymaktivität zu stoppen. Der pH-Wert nach dem erneuten Säurezusatz beträgt ca. 4.

Die so erhaltene Suspension wird dann durch ein Haarsieb abgeseiht und die erhaltene Lösung im Vakuum (40 Torr) eingeengt oder sprühgetrocknet.

Der erhaltene Farbstoff hat in 0,1%iger wässriger Lösung (pH 4) eine kräftig rote Färbung mit leichtem Lilastich.

Der sprühgetrocknete Farbstoff ist schwarzviolett gefärbt.

Beispiel 2

Beispiel 1 wurde mit dem Schalenstaub, der als Nebenprodukt beim Entschälen von Bohnen zur Herstellung von weissem Bohnenmehl anfällt, wiederholt.

Bei Verwendung von 250 g Schalenstaub wurde identisch wie in Beispiel 1 gearbeitet, jedoch wurden die 250 g Schalenstaub mit einem Gemisch aus 1 l Wasser und 0,5 g pulverisierter kristallierter Zitronensäure 10 Minuten lang gekocht und dann wie in Beispiel 1 beschrieben weiterverarbeitet. Es ergab sich der gleiche Farbstoff. Es wird praktisch der gesamte Farbstoff aus den Schalen entfernt, wenn der Zellaufschluss hinreichend durchgreifend ist. Bei Verwendung von Zitronensäure beträgt die Ausbeute ca. 2 bis 2,5% des eingesetzten Schalengewichts.

Die Wiederholung von Beispiel 1 mit Ascorbinsäure anstatt Zitronensäure in der gleichen Menge ergab eine Ausbeute von 1,8 bis 2,0%.

Beispiel 3

2000 g schwarze Bohnen werden mechanisch geschält, wobei 500 g Schalen gewonnen werden, die für das weitere Verfahren benötigt werden. Das rückständige Bohnenmus wird abgesondert und steht für anderweitige Verwendung zur Verfügung. Beim Schälen wird auch noch die oberste, deutlich gefärbte Schicht des Bohnenkernes selbst mit abgeschliffen. Zum Schälen wird eine handelsübliche Labormaschine, wie sie für das Schälen von Hülsenfrüchten bekannt sind, eingesetzt.

Die erhaltenen 500 g Schalen werden mit 2,5 l Wasser angesetzt und diese wässrige Suspension mit einer Oxycarbonsäure behandelt, wobei der pH-Wert auf 4 eingestellt wird. Der Autoklaviervorgang wird über einen Zeitraum von einer Stunde bei einem Druck von 150 bar und einer Temperatur von 80°C durchgeführt. Die weitere Verarbeitung erfolgt wie im Beispiel 1. Die Ausbeute beträgt ca. 2%. Als Oxysäure wurde bei einem Versuch Zitronensäure, bei einem weiteren Versuch Ascorbinsäure und bei einem dritten Versuch Weinsäure verwendet. Es ergaben sich praktisch die gleichen Ergebnisse.

Die folgenden Beispiele erläutern die Anwendung des Farbstoffes:

I – Hartbonbons
| | | |
|---|---|---|
| 8,58 | l | Wasser |
| 26,0 | kg | Zucker |
| 8,0 | kg | Glucose (43–45°Be) |
| 0,375 | kg | Zitronensäure |
| 0,037 | kg | Aroma |
| | | Lebensmittelfarbstoff |
| 0,400 | kg | (5%ig gelöst in Wasser) |
| 43,40 | | |

II – Wassereis
| | | |
|---|---|---|
| 2,5 | kg | Bindemittelmischung |
| 30,0 | kg | Kristallzucker |
| 5,0 | kg | Stärkesirup 43°Be |
| 61,615 | kg | Wasser |
| 0,035 | kg | Zitronensäure |
| 0,850 | kg | Farbstofflösung (3%ig in Wasser) |
| 100,000 | kg | Mischung |

III – Limonadengetränk
| | | |
|---|---|---|
| 13,32 | l | Zuckersirup 65%ig |
| 0,058 | l | Benzoesäure 30%ig |
| 0,072 | l | Zitronensäure 50%ig |
| 0,100 | kg | Aroma |
| 0,850 | l | Farbstofflösung 2,5 Gew.-%ig in Wasser |

IV – Bitter Aperitif (alkoholhaltig) Typ «Campari»
| | | |
|---|---|---|
| 29,5 l | Sprit 96 Vol. % | |

| | | |
|---|---|---|
| 43,1 | l | Wasser |
| 22 | l | Zuckerlösung 65%ig |
| 3,0 | l | Cocktail-Bitter-Essenz |
| 0,8 | l | Zitronensäure 50%ig |
| 4 | l | Farbstoff 5%ig, gelöst in Wasser |
| 102,4 | l | |
| 2,4 | l | Kontraktion |
| 100,0 | l | Fertigware |

V – Weingummi

1.
| | |
|---|---|
| 75,00 g | Gelatine, 180° Bloom |
| 5,00 g | Agar |
| 210,00 g | Wasser |

2.
| | |
|---|---|
| 68,00 g | Wasser |
| 290,00 g | Kristallzucker |
| 330 g | Glucosesirup, 45°Be |
| 10,00 g | Glycerin |
| 35,00 g | Zitronensäure 50%ig in Wasser |
| 12 g | Farbstofflösung 5%ig in Wasser |
| 1035,00 g | |

Arbeitsanleitung:

Zu 1.: 5,0 g Agar in 210,0 g Wasser lösen und kurz aufkochen. Lösung auf 75°C abkühlen und 75,0 g Gelatine zusetzen.

Zu 2.: 290,0 g Kristallzucker in 80,0 g Wasser lösen und aufkochen.

330,0 g Glucosesirup zugeben und auf 118°C kochen.

Die Kochlösung abkühlen auf 70°C. Dann Gelatine-Agar-Lösung zugeben und 10,0 g Glycerin.

Bei 65°C Temperatur der Weingummimasse werden die angegebene Menge Säure und Farbe und nach Belieben Aroma untergemischt.

Ausführungsbeispiele

VI – Schaschliksauce (Tomatenketchup)
| | |
|---|---|
| 8,0 g | Citronensäure |
| 10,0 g | Senf (mittelscharf) |
| 25,0 g | Kochsalz |
| 30,0 g | Stärke 3818 |
| 100,0 g | Essig 10% |
| 100,0 g | Glukosesirup |
| 130,0 g | Zucker |
| 8,5 g | Farbstofflösung (3%ig in Wasser) |
| 588,5 g | Wasser |
| 1000,0 g | |

Der Einsatz des erfindungsgemässen Farbstoffes ermöglicht die Einsparung von Tomatenmark. Farbstoff und Tomatenmark können aber beliebig gemischt werden.

VII – Salatmayonnaise (Dressing)
| | |
|---|---|
| 1,0 % | Kochsalz |
| 1,5 % | Zucker |
| 2 % | Senf |
| 5 % | Essig 10%ig |
| 5,5 % | Mayomil M (Fa. Schmidt) |
| 6 % | Eigelb |
| 28,15 % | Wasser |

0,85 % Farbstoff (3%ig in Wasser)
50 % Speiseöl
100 %

VIII – Fruchtzubereitung
0,4 % Frimulsion IQU
30 % Frucht
42 % Zucker
12 % Glucosesirup
0,4 % Natriumzitrat
15,2 % Wasser
100 %

Die so hergestellte Fruchtzubereitung wird je nach Verwendungszweck mit dem erfindungsgemässen Farbstoff eingefärbt.

IX – Papier

In einer 1%igen wässrigen Lösung, die vorzugsweise einen pH-Wert von 4,5 aufweist, wird das Papier durch einmaliges Eintauchen und anschliessende Trockung gefärbt. Dies ergibt einen intensiv roten Farbton. Farbtonänderungen können durch Verschiebung des pH-Wertes bewirkt werden.

**Patentansprüche**

1. Natürlicher geniessbarer Farbstoff, insbesondere für die Farbgebung von Lebens- und Genussmitteln, Getränken, pharmazeutischen und kosmetischen Erzeugnissen, gekennzeichnet durch das IR-Spektrum gemäss Figur 1 und das UV-Spektrum gemäss Figur 2.

2. Verfahren zur Herstellung des Farbstoffes nach Anspruch 1, dadurch gekennzeichnet, dass man die Zellen der Schalen farbiger Bohnen der Gattung Phaseolus, insbesondere schwarzer Bohnen, zerstört, den freigesetzten Farbstoff, ggf. nach schwachem Ansäuern, mit Wasser oder einer wässrigen Lösung einer schwachen organischen Säure extrahiert, den erhaltenen Extrakt von Schalenresten trennt und die Lösung in an sich bekannter Weise konzentriert und ggf. trocknet.

3. Verfahren nach Anspruch 2, dadurch gekennzeichnet, dass man die Zerstörung der Zellen durch Behandlung mit einem geeigneten Enzym durchführt.

4. Verfahren nach Anspruch 2, dadurch gekennzeichnet, dass man die Extraktion mit der wässrigen Lösung einer Oxycarbonsäure durchführt.

5. Verfahren nach Anspruch 4, dadurch gekennzeichnet, dass man die Extraktion mit wässriger Zitronensäure durchführt.

6. Verfahren nach Anspruch 2 oder 3, dadurch gekennzeichnet, dass man die Bohnenschalen zur Vorbereitung für die Zellzerstörung mit verdünnter wässriger saurer Lösung, insbesondere Lösungen einer organischen Säure, kocht und gegebenenfalls auf den für die enzymatische Zellzerstörung geeigneten pH-Wert und die geeignete Temperatur bringt.

7. Verfahren nach Anspruch 2 oder 3, dadurch gekennzeichnet, dass man nach der Zellzerstörung und vor dem Konzentrieren weitere Säure zusetzt.

8. Verfahren nach Anspruch 3, dadurch gekennzeichnet, dass man als Enzym Säureamylase verwendet.

9. Verfahren zur Herstellung des Farbstoffes nach Anspruch 1, dadurch gekennzeichnet, dass man

a) die Schalen von farbigen Bohnen der Gattung Phaseolus oder deren Pulver mit so viel schwach saurem Wasser, dass eine gut rührfähige Suspension entsteht, einige Zeit kocht, wobei der pH-Wert nach 10 bis 15-minütigem Kochen etwa 5 bis 5,5 betragen soll,
b) nach Abkühlen auf eine, für die nachstehend angegebene Verfahrensstufe zweckmässige Temperatur die Zellwände der Bohnenschalen in an sich bekannter Weise durch enzymatischen Abbau oder Vermahlen bei tiefen Temperaturen oder durch Autoklavieren unter erhöhtem Druck und erhöhter Temperatur zwecks Freisetzung des Farbstoffes wenigstens teilweise zerstört,
c) die erhaltene Suspension durch Zusatz einer schwachen organischen Säure wieder auf einen pH-Wert von etwa 4 bis 4–5 bringt,
d) die gemäss c) erhaltene Suspension in an sich bekannter Weise in Schalenrückstände und Farbstofflösung trennt und
e) die Farbstofflösung in an sich bekannter Weise, ggf. bis zur Trockne, einengt.

10. Verfahren nach Anspruch 2, dadurch gekennzeichnet, dass man den Farbstoff sprühtrocknet.

11. Verwendung des Farbstoffs nach Anspruch 1 zur Färbung von Textilien, Papier und Leder.

**Claims**

1. Natural edible dyestuff, in particular for colouring foodstuffs and stimulants, beverages, pharmaceutical and cosmetic products, characterised by the IR spectrum according to Figure 1 and the UV spectrum according to Figure 2.

2. Process for the preparation of the dyestuff according to Claim 1, characterised in that the cells of the hulls of coloured beans of the genus Phaseolus, in particular black beans, are destroyed, the liberated dyestuff is extracted, if appropriate after weak acidification, with water or an aqueous solution of a weak organic acid, the extract obtained is separated from the hull residues and the solution is, in a manner known in itself, concentrated and, if appropriate, dried.

3. Process according to Claim 2, characterised in that the destruction of the cells is carried out by treatment with a suitable enzyme.

4. Process according to Claim 2, characterised in that the extraction is carried out with the aqueous solution of a hydroxycarboxylic acid.

5. Process according to Claim 4, characterised in that the extraction is carried out with aqueous citric acid.

6. Process according to Claim 2 or 3, characterised in that, in preparation for the destruction of the cells, the bean hulls are boiled with a dilute aqueous acid solution, in particular solutions of an organic acid, and, if appropriate, the pH and the temperature are adjusted to a value suitable for the enzymatic cell destruction.

7. Process according to Claim 2 or 3, characterised in that further acid is added after the cell destruction and before concentrating.

8. Process according to Claim 3, characterised in that the enzyme used is acid amylase.

9. Process for the preparation of the dyestuff according to Claim 1, characterised in that

a) the hulls of coloured beans of the genus Phaseolus, or a powder thereof, is boiled for some time with such a quantity of weakly acid water that a suspension is formed which can readily be stirred, the pH value being intended to be about 5 to 5.5 after boiling for 10 to 15 minutes,
b) after cooling to a temperature appropriate for the process step indicated below, the cell walls of the bean hulls are at least partially destroyed, in a manner known in itself, by enzymatic degradation or by grinding a low temperatures or by autoclaving under an elevated pressure and at an elevated temperature, in order to liberate the dyestuff,
c) the suspension obtained is brought again to a pH value of about 4 to 4.5 by adding a weak organic acid,
d) the suspension obtained according to c) is separated, in a manner known in itself, into hull residues and dyestuff solution and
e) the dyestuff solution is concentrated in a manner known in itself, if appropriate to dryness.

10. Process according to Claim 2, characterised in that the dyestuff is spray-dried.

11. Use of the dyestuff according to Claim 1 for colouring textiles, paper and leather.

**Revendications**

1. Colorant alimentaire naturel, notamment pour colorer des produits alimentaires et produits stimulants, des boissons, des produits pharmaceutiques et cosmétiques, caractérisé par le spectre infrarouge suivant la figure 1 et le spectre ultraviolet suivant la figure 2.

2. Procédé de production du colorant suivant la revendication 1, caractérisé en ce qu'on détruit les cellules des enveloppes de haricots de couleur du genre Phaseolus, notamment des haricots noirs, on extrait le colorant libéré, par exemple après faible acidification, avec de l'eau ou avec une solution aqueuse d'un acide organique faible, on sépare l'extrait obtenu des restes d'enveloppes et on concentre et, le cas échéant, on déshydrate la solution d'une manière connue.

3. Procédé suivant la revendication 2, caractérisé en ce qu'on conduit la destruction des cellules par traitement avec un enzyme approprié.

4. Procédé suivant la revendication 2, caractérisé en ce qu'on conduit l'extraction avec la solution aqueuse d'un acide oxycarboxylique.

5. Procédé suivant la revendication 4, caractérisé en ce qu'on conduit l'extraction avec l'acide citrique aqueux.

6. Procédé suivant la revendication 2 ou 3, caractérisé en ce qu'on fait bouillir les enveloppes de haricots, en préliminaire à la destruction des cellules, avec une solution acide aqueuse diluée, notamment des solutions d'un acide organique et, le cas échéant, on les ajuste à la valeur de pH appropriée pour la destruction enzymatique des cellules et à la température appropriée.

7. Procédé suivant la revendication 2 ou 3, caractérisé en ce qu'on ajoute de l'acide après la destruction des cellules et avant la concentration.

8. Procédé suivant la revendication 3, caractérisé en ce qu'on utilise une amylase acide comme enzyme.

9. Procédé de production du colorant suivant la revendication 1, caractérisé en ce que

a) on fait bouillir un certain temps les enveloppes de haricots de couleur du genre Phaseolus ou leur poudre avec suffisamment d'eau légèrement acide pour obtenir une suspension facile à agiter, le pH devant alors s'élever à environ 5–5,5 après ébullition pendant 10 à 15 minutes,
b) après refroidissement à une température qui convient à la mise en œuvre de l'étape du procédé indiquée ci-après, on détruit au moins partiellement les parois des cellules des enveloppes de haricots d'une manière connue par dégradation enzymatique ou par broyage à basses températures ou par autoclavage sous pression et à température élevées en vue de libérer le colorant,
c) on ajuste de nouveau à une valeur d'environ 4 à 4–5 le pH de la suspension obtenue, par addition d'un acide organique faible,
d) on divise d'une manière connue la suspension obtenue conformément à c) en résidus d'enveloppes et en solution de colorant et
e) on concentre éventuellement à sec d'une manière connue la solution de colorant.

10. Procédé suivant la revendication 2, caractérisé en ce qu'on sèche le colorant par pulvérisation.

11. Utilisation du colorant suivant la revendication 1 pour la teinture de matières textiles, de papier et de cuir.

FIG.1

FIG. 2

$\lambda max \approx 518\ nm$

$\varepsilon' \approx 1,08\ cm^2/mg$

300    500    700

0 054 562

| SAMPLE Lebensmittelfarbstoff | NOTES mit Zitronensaüre | MODE A B S | SCALE HIGH/LOW 2 / 0 |
|---|---|---|---|
| ORIGIN | gepuffert | DERIVATIVE | RESPONSE 1 |
| CONCENTRATION c'= 1 mg/ml | | CONC FACT | FORMAT |
| PATHLENGTH s = 1 cm | | BACKGROUND CORR. | SCAN SPEED 3 0 nm/min |
| SOLVENT Dest. Wasser | | SLIT 2,0 | CYCLE TIME min |